# EUROPEAN PATENT APPLICATION

(11) **EP 3 845 833 A1**
(43) Date of publication of application: **07.07.2021**
(21) Application number: 20174714.4
(22) Date of filing: 14.05.2020
(51) Int. Cl.: F25D 17/00

(54) **APPARATUS FOR THE PRESERVATION OF FRESH ITEMS**

(30) Priority: 30.12.2019 GB 201919413
(71) Applicant: Seven Seas Productions Ltd, Mayfair London W1J 6HE (GB)
(72) Inventor: Girardin, Maxime, London, W1J 0DW (GB); Montague, Jonathan Wesley, London, W1J 0DW (GB)
(74) Representative: Hocking, Adrian Niall

(57) **Abstract**

An apparatus is provided for preservation of fresh items or produce. The apparatus comprises a closed housing (1) to enable an environment within the housing to be controlled, a shelf (3) within the housing (1) for supporting fresh items, an ozone generator (10), a humidifier (8) and a propagation or distribution system (24, 17). The propagation or distribution system (24, 17) is configured to transfer ozone generated by the ozone generator (10) and water particles produced by the humidifier (8) separately to the vicinity of the shelf (3) before releasing both the ozone and water particles into the housing (1).

## Description

The present invention relates to an apparatus for the preservation of fresh items or produce such as vegetables, fruits, herbs and berries as well as flowers and any other perishable goods such as meat and fish.

Every year more than US$680 billion worth of food is wasted in industrialized countries. From these amounts, 40 to 50% is attributable to fresh food. This is mostly due to the short-shelf life of most fresh produce. Fresh vegetables and fruit tend to spoil rapidly as soon as they have been harvested. The main threats for fresh produce are oxygen in the form of dioxygen, high temperature and low relative humidity.

Some storage facilities use ozone to increase the shelf life of produce by killing any pathogen developing on the skin of the produce. It is already used in some storage areas for some types of produce.

Increasing humidity in a storage facility can extend slightly the shelf life of produce by preventing the produce from drying out since the drying out would cause the consumption of the vitamins of the produce.

Some storage facilities use ozone and adjustment of humidity in combination. When used together, they are either propagated through the same pipe or from the same blowing outlet. This results in some produce receiving an overdose of ozone and therefore damaging the produce, or not receiving ozone and therefore not improving the shelf-life. The same applies for the humidity. Delivering the right amount of ozone and humidity is critical as both ozone and humidity can either damage the product or not have any effect at all if not delivered at the right dose.

It is an object of the present invention to provide an apparatus to alleviate at least one of the above-mentioned problems.

According to one aspect of the present invention there is provided an apparatus for preservation of fresh items or produce, said apparatus comprising a closed housing to enable the environment within the housing to be controlled, a shelf within the housing for supporting fresh items, an ozone generator, a humidifier and a propagation or distribution system for transferring ozone generated by the ozone generator and water particles produced by the humidifier separately to the vicinity of the shelf before releasing both the ozone and water particles into the housing.

The housing preferably contains a plurality of shelves for supporting fresh items, and the propagation or distribution system preferably transfers or is configured to transfer ozone and water particles separately to each shelf for release in the vicinity of each shelf.

In a preferred embodiment, the propagation or distribution system comprises a plurality of pipes extending from the ozone generator and the humidifier to the or each shelf. The plurality of pipes may comprise first and second main pipes connected at one end thereof to the ozone generator and humidifier respectively. Each main pipe may have a plurality of subsidiary pipes branching from its other end to distribute ozone and water particles in the vicinity of the shelf. The apparatus may have a closed loop air recirculation system including air inlet conduits connected to a cooling system for cooling recirculated air. The first main pipes may be connected to the ozone generator and cooling system.

A weighing mechanism may be provided on the or each shelf to weigh the items stored on the shelf and to detect a change in weight when one or more items are removed from the shelf. The weighing mechanism may be connected to a printer to enable the weight and/or price of items removed from the shelf to be recorded.

The apparatus preferably includes an automated control system for controlling operation of the humidifier, the ozone generator and the cooling system. The or each shelf may have at least one of a humidity sensor, an ozone sensor and a temperature sensor connected to the control system, wherein the control system is arranged to adjust automatically the operation of the humidifier, the ozone generator, and the cooling system in response to signals from said sensor or sensors. This may provide the environment required in the closed cabinet. The apparatus may include an infrared camera to assess the level of ethylene emitted from the items to control the levels of humidity, ozone and/or temperature.

The humidifier may be an ultrasonic humidifier. It may be connected to a water tank for containing water to be supplied to the humidifier. The water tank may be slidable from the cabinet to outside of the closed housing to enable it to be refilled with water. The apparatus may include a reverse osmosis system to purify the water supplied to the humidifier.

The closed housing preferably has a display or storage area containing the one or more shelves and a support area separate from the display area in which the humidifier, cooling system and ozone generator are located. The display area is accessed through at least one door, which is preferably self-closing.

The or each shelf may be angled with respect to the horizontal to allow drainage of water from the items on the shelf.

The apparatus may include ultra-violet (UV) lighting to illuminate fresh items stored on the or each shelf.

According to another aspect of the invention, there is provided a method for preserving fresh items comprising the steps of:-
storing the fresh items on one or more shelves in a closed housing;
feeding ozone and humidity separately from each other to the vicinity of the or each shelf; and
releasing the ozone and humidity into the closed housing in said vicinity.

The method may also include one or more of the steps of:-
weighing items stored on the or each shelf so as to detect a change in weight when one or more items are removed from the shelf;
circulating cooled air around the interior of the closed housing;
sensing at least one of the humidity, ozone and temperature levels at the or each shelf and automatically adjusting the levels to provide a desired environment in the closed housing;
automatically changing the levels of humidity and ozone released in the vicinity of the or each shelf in accordance with determined time periods and/or times of day and night;
illuminating the fresh items stored on the or each shelf with UV lighting;
using an infra-red camera to assess the level of ethylene being emitted by the items stored on the or each shelf to adjust the required levels of humidity, ozone and/or temperature in the vicinity of the shelf.

By keeping the ozone and humidity separate, the concentration of ozone suffers less disintegration (i.e. return to an oxygen form) from the humidity prior to reaching the produce. Therefore, a much higher propagation rate of ozone can be achieved without having to generate more ozone.

By having a propagation or distribution system using separate main pipes in combination with smaller subsidiary pipes located directly on each shelf, a homogenous and optimised propagation or distribution level of both ozone and humidity for all the produce stored in the cabinet is guaranteed.

The apparatus provides a closed environment which is required to provide the conditions to significantly extend fresh produce shelf life without the use of chemicals.

The apparatus may comprise at least part of a standalone display cabinet designed to store fresh vegetables and fruit. The apparatus may be used by retailers, and in kitchens, restaurants and hotels, for example. The apparatus can have a wide range of size, design and shape so the apparatus can be used for storing different products, such as vegetables, fruits, herbs and flowers, in a wide variety of locations.

The closed environment provided by the apparatus dramatically increases the shelf-life of fresh produce. The apparatus may also preserve the fresh visual appearance of the stored produce and their nutritional quality for much longer. The relative humidity, ozone level and/or temperature may be closely monitored and controlled to increase shelf-life and produce quality, and provide optimal conditions for this. Specific temperature, humidity, ozone and/or light levels may be used. The apparatus can extend the shelf life of fresh food products by a factor of up to three. The produce would have a fresh visual appearance for longer.

Use of the apparatus to preserve fresh produce for longer would enable a significant decrease in fresh food wastage which reduces land requirements, water required for irrigation, chemical use, and production carbon footprint. Methane production in landfills would also be reduced. It would also significantly reduce economic losses for fresh food retailers and end-users. Less waste means that fewer deliveries are required which reduces the carbon footprint of transport, and reduces traffic in areas of habitation such as cities. Less waste also means less packaging is required which can reduce use of plastics. The decrease in fresh food wastage generates significant revenues for retailer as less restocking is required. Being able to sell fresh produce for longer could improve the reputation of the retailer.

The delivery of ozone by the apparatus lowers the risk of contamination of food stored in the apparatus by oxidizing or suppressing micro-organism development, including but not limited to pathogens, bacteria and fungi, leading to safer food. The apparatus may include UV lighting to suppress micro-organism development.

The applied humidity preserves vitamin content by stopping the drying process and the metabolism of the produce leading to healthier food. The applied humidity also enables the weight of the produce to be maintained wherein the weight is significantly affected by the low relative humidity or dryness of an environment.

The increased preservation of food by the apparatus removes chemicals needed to be applied to preserve food leading to healthier and safer food, and food which tastes better.

Using an ultrasonic humidifier means that the produce has condensation on it but the produce is not soaked.

The apparatus may have an automated timing system in relation to control operation of the ozone generator. Thus, the ozone generator could be arranged to only produce a maximum concentration of ozone in the apparatus at night to prevent any potential human contamination from the ozone. The automated timing system may be used to control operation of the humidifier.

Embodiments of the present invention will now be described, by way of example, with reference to the accompanying schematic drawings, in which:
Figure 1 is a front view of an apparatus in the form of a display cabinet in accordance with a first embodiment of the present invention;
Figure 2 is a front view of the apparatus with doors omitted;
Figure 3 is a sectional view taken along lines 3-3 of Figure 1;
Figure 4 is a rear view of the apparatus with a back cover removed and a cooling air/ozone system omitted;
Figure 5 is a rear view of part of the apparatus with the back cover removed and including the cooling air/ozone system;
Figure 6 is a diagrammatic cross-sectional view of part of the apparatus;
Figure 7 is a diagrammatic longitudinal sectional view of part of the apparatus;
Figure 8 is a plan view of a shelf of the apparatus;
Figure 9 is a view of a weighing pad forming part of the shelf;
Figure 10 are views of control systems for part of the apparatus;
Figure 11 is a front view of an apparatus in the form of a home refrigerator in accordance with a second embodiment of the present invention;
Figure 12 is a front view of the apparatus of Figure 11 with part of the door omitted;
Figure 13 is a side view of an apparatus in the form of a transportation container in accordance with a third embodiment of the present invention; and
Figure 14 is a view of piping networks to one side of the apparatus of Figure 13.

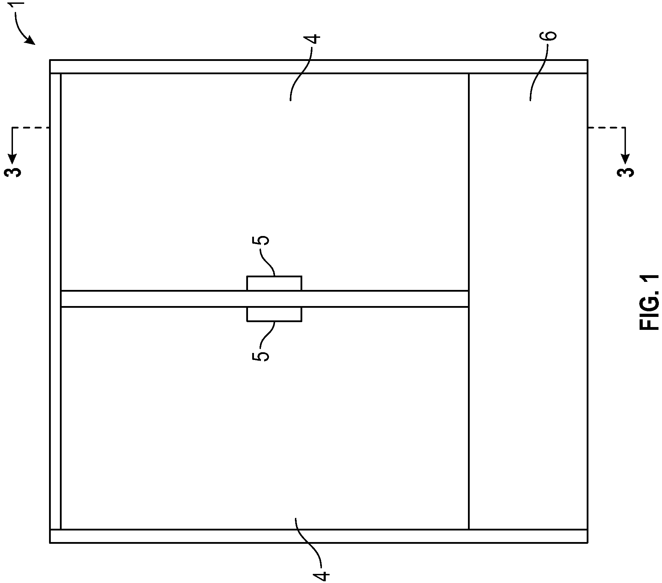

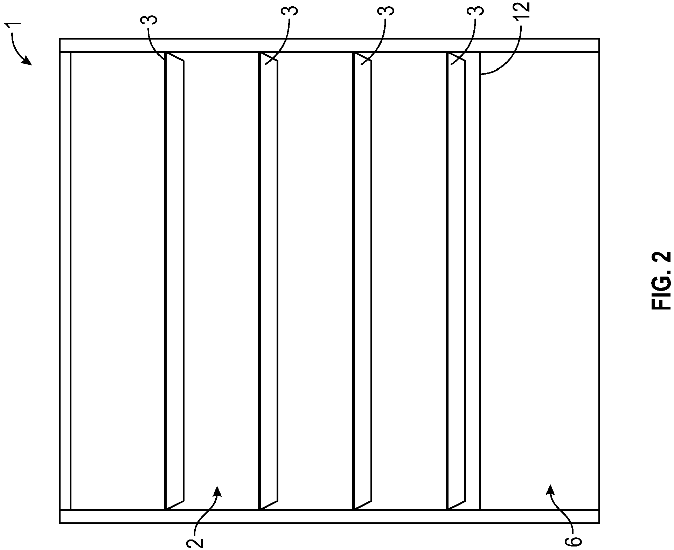

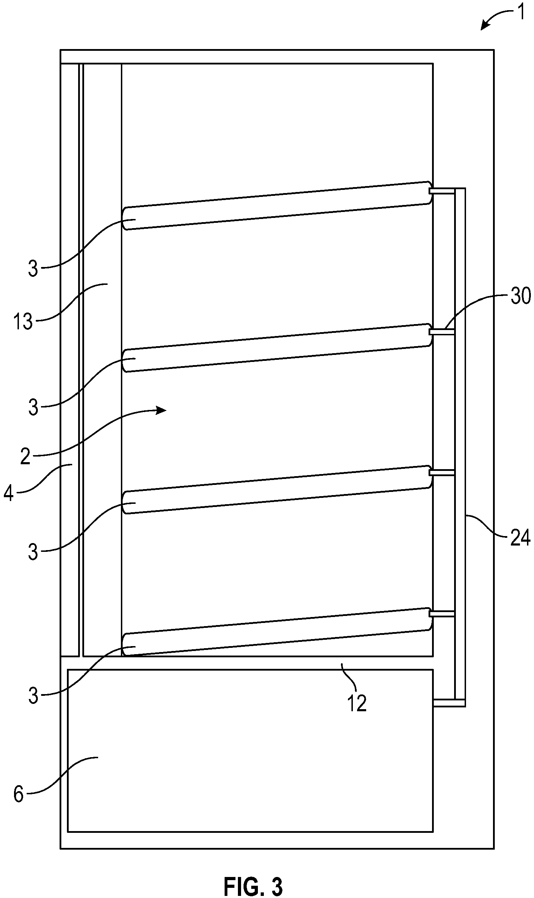

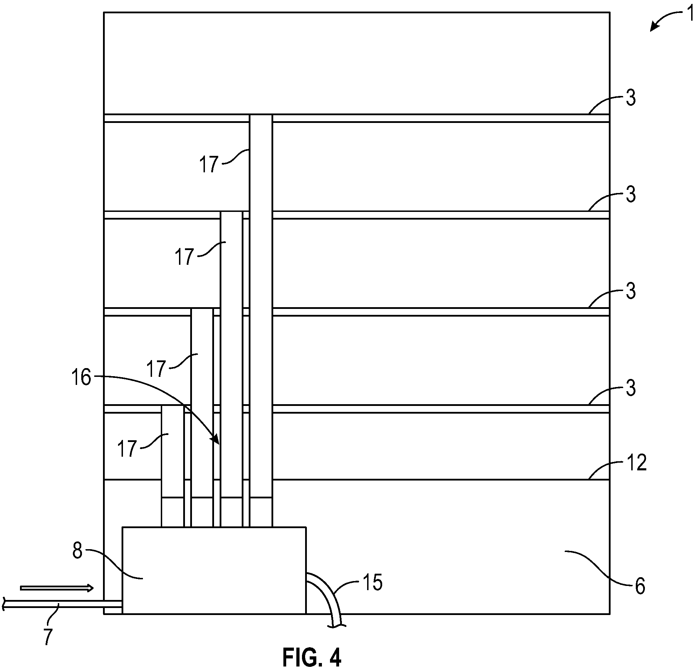

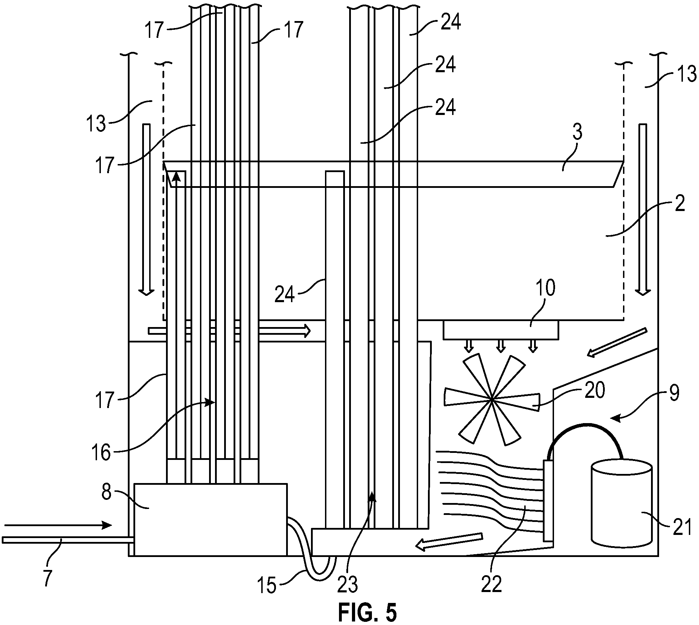

Referring to Figures 1 to 8 of the accompanying drawings, an apparatus or pod 1 according to a first embodiment of the invention is shown in the form of a stand-alone display cabinet or closed housing designed to store fresh vegetables and fruits. The pod 1 has a display or storage area 2 containing several shelves 3 for storing fresh produce. The display area 2 forms a controlled internal environment that can extend the shelf life of fresh food products or produce. The controlled environment includes the maintenance of specific humidity, ozone levels, and temperature.

The display area 2 has display lights (not shown) to illuminate produce being displayed in the cabinet 1.

The display area 2 is accessed through two doors 4 on the front of the cabinet 1 that open outwards or that can be slid by use of each handle 5 on the door 4. The doors 4 are self-closing to prevent the ozone, humidity and cool air from dissipating from the inside of the cabinet 1. The doors 4 have an anti-UV film on their outer side to prevent natural UV light from entering the cabinet 1 and accelerating the degradation process. The doors 4 also have a hydrophobic coating on the internal side to prevent the condensation and accumulation of water. This helps maintain the cleanliness of the display area 2 while improving the customer's interaction as the cabinet 1 looks cleaner. The doors 4 are transparent and may comprise glass, plexiglass or any other suitable transparent material.

Within the base of the cabinet 1 is a support area or section 6 having a water inlet 7, an ultrasonic humidifier 8, a cooling system 9, and an ozone generator 10 which are used for control and maintenance of the internal environment, together with an automated control system 11 for controlling the humidifier 8, cooling system 9 and ozone generator 10.

The display area 2 is separated from the support section 6 by a sheet 12, which may be a welded sheet of metal or a clipped sheet of plastic, to isolate fresh produce in the display area 2 from the support section 6.

All faces of the cabinet 1 except for the part of the front face having the transparent doors 4 are closed and opaque, fitted with insulation material. The base of the cabinet 1 is also covered to hide and protect the equipment in the support section 6. The front section of the support section 6 can be opened to provide access the different equipment located in the support section 6 for maintenance and for re-filling.

The cabinet 1 has inner and outer shells between which there is a layer of insulating medium used to limit the waste of temperature due to the external environment. The inner shell separates the insulating medium from the fresh produce in the display area 2.

Located just behind the doors 4 on either side of the display area 2 is an internal air inlet conduit 13 forming part of an air recirculating system 14 for recirculating the air inside the cabinet 1. The air recirculating system 14 operates as a closed loop system where the air inlet conduits 13 are connected to the cooling system 9 in the support section 6. Each air inlet conduit 13 comprises perforations in the side of the display area 2 which lets air enter the inlet conduit 13 whilst preventing parts of produce (e.g. leaves) from entering the air recirculating system 14.

The ultrasonic humidifier 8 is directly connected to a water network by the water inlet 7 which may comprise a hose. The water network supplies demineralized water to the ultrasonic humidifier 8. The water network may include a water tank in the support section 6, and the water tank may be mounted on slides so that it can be easily moved for operation and maintenance purposes. The ultrasonic humidifier 8 is connected to water disposal piping 15 with valve control for evacuating excess water from the humidifier 8. The piping and hose 7 are made of polymer type material that is certified for use with potable water. The ultrasonic humidifier 8 has a built-in water filtration system and at least one blower or fan.

Moisture and humidity progress from the ultrasonic humidifier 8 to a main humidity distribution system 16. The distribution system 16 is made of polymer or similar material that is inert to moisture. The main humidity distribution system 16 comprises a plurality of flexible main distribution pipes or exhaust tubes 17 at the back of the cabinet 1 wherein each pipe 17 connects the humidifier 8 to a humidity piping network 19 in a respective shelf 3 for propagation on the produce. Each flexible pipe 17 is L-shaped and extends out of the humidifier 8 before extending upwards to the shelf humidity piping network 19. There may be flexible main humidity distribution pipes 17 in each rear corner of the display area 2.

Air in the display area 2 enters the air inlet conduit 13 on either side of the display area 2 and the air inlet conduits 13 are connected to the cooling system 9 in the support section 6. Some air enters the cooling system 9 via the ozone generator 10, which is electrically powered, and the ozone generator 10 enriches the air with ozone. The ozone generator 10 contains a blower to propagate ozone into the air which is being recirculated. A powerful fan 20 is located downstream of the air inlet conduits 13, the ozone generator 10 and upstream of the cooling system 9. The fan 20 is used to suck in the used air from the display area 2 to the cooling system 9. The cooling system 9 comprises a compressor 21 used to refrigerate gas in a condenser 22 of the system 9. The condenser 22 is in direct contact with the recirculated air and cools it down.

The water disposal piping 15 with valve control that leads from the humidifier 8 also has a branch coming from the condenser 22 and is used to evacuate excess water from the cabinet 1.

The cooled air enriched in ozone is then constrained to go into a main cool air and ozone distribution system 23 by positive pressure generated by the fan 20. The main cool air and ozone distribution system 23 comprises a plurality of flexible main distribution pipes 24 at the back of the cabinet 1 wherein each pipe 24 connects the cooling system 9 to a cool air and ozone piping network 25 in a respective shelf 3 for propagation on the produce. Each flexible pipe 24 is L-shaped and extends out of the cooling system 9 before extending upwards to the shelf cool air and ozone piping network 25. There may be flexible main cool air and ozone distribution pipes 24 in each rear corner of the display area 2.

An outside air inlet 26 with a pump is connected to the main cool air and ozone distribution system 23 for pumping in air from outside the cabinet 1 if required.

The sheet 12 separating the display area 2 from the support section 6 has holes for the air inlet conduits 13, the main humidity distribution pipes 17 and the main cool air and ozone distribution pipes 24 to pass through.

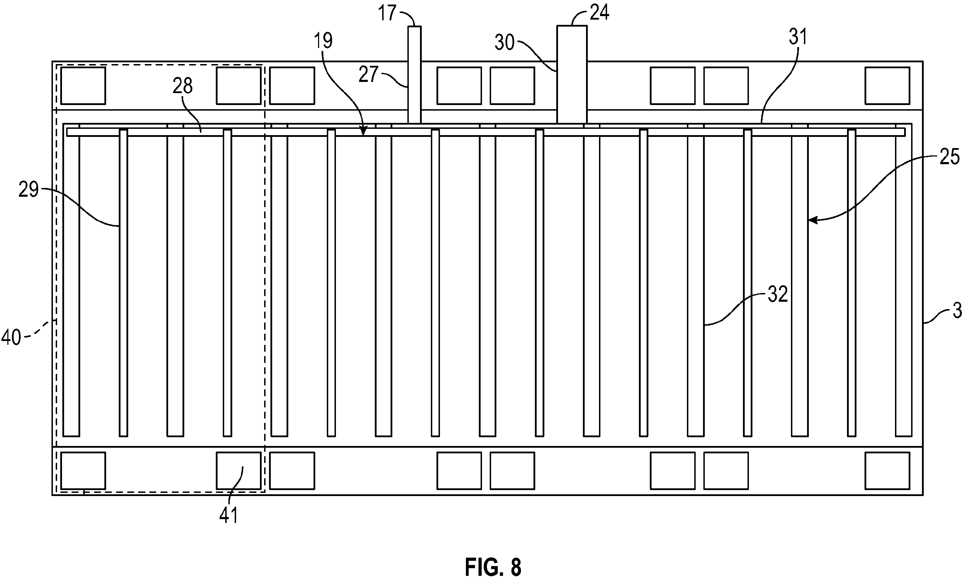

Referring particularly to Figure 8, the shelf humidity piping network 19 in each shelf 3 has a connection pipe 27 (which may be L-shaped) connecting the main humidity distribution pipe 17 extending to that shelf 3 to a shelf humidity distribution pipe 28 extending along the back of the shelf 3. A series of branches or subsidiary pipes 29 extend perpendicularly to the shelf humidity distribution pipe 28 across the shelf 3 towards the front of the shelf 3.

The shelf cool air and ozone piping network 25 in each shelf 3 has a connection pipe 30 (which may be L-shaped) connecting the main cool air and ozone distribution pipe 24 extending to that shelf 3 to a shelf cool air and ozone distribution pipe 31 extending along the back of the shelf 3. A series of branches or subsidiary pipes 32 extend perpendicularly to the shelf cool air and ozone distribution pipe 31 across the shelf 3 towards the front of the shelf 3. In each shelf 3, the shelf cool air and ozone distribution pipe 31 and branches 32 are located above and staggered with respect to the shelf humidity distribution pipe 28 and branches 29.

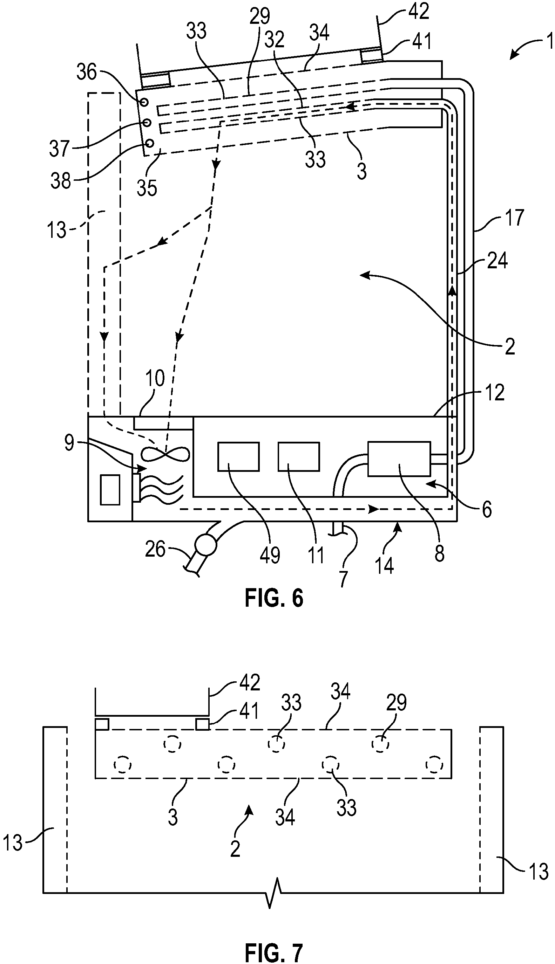

Each branch 29, 32 has a series of holes 33 (see Figures 6 and 7) along its top and bottom surface with corresponding holes 34 in upper and lower surfaces of the shelf 3 for the release of agents and gases both above and below the shelf 3 for a homogenous propagation. The pipes 17, 27, 28, 24, 30, 31 and branches 29, 32 are made of polymers and designed to be inert to ozone and humidity.

Each shelf 3 has an angle of tilt down towards the front of the cabinet 1 to enable surplus water in the shelf 3 to drain from the shelf 3 via a drain opening 35. This prevents water accumulation that could enable bacteria, germs and micro-organisms to develop.

Each shelf 3 has a humidity sensor 36, an ozone sensor 37 and a temperature sensor 38 which are connected to the control system 11. Depending upon the readings received from these sensors 36, 37, 38, the control system 11 can automatically adjust the operation of the ultrasonic humidifier 8 and the cooling system 9 to provide the environment required in the display area 2.

A mist or fog of cold fine water droplets or nano-water particles from the ultrasonic humidifier 8 is diffused from the holes 33 in the humidity piping network branches 29 in the shelves 3 to the display area 2. These droplets do not heat up the produce and do not wet the produce to any significant extent. At least some condensation in the display area 2 may be collected and recirculated to the ultrasonic humidifier 8.

The shelves 3 are made of metal sheets 39 that can hold the weight of the produce on display. The upper surface of each shelf 3 has groups 40 of four pads or pods 41 wherein each pad 41 of the group 40 is arranged to support a corner of a basket 42 for displaying loose fresh produce. The pads 41 of each group 40 will be used in the automated weighing process. The pads 41 of each group 40 are designed to weigh the basket 42 at all times.

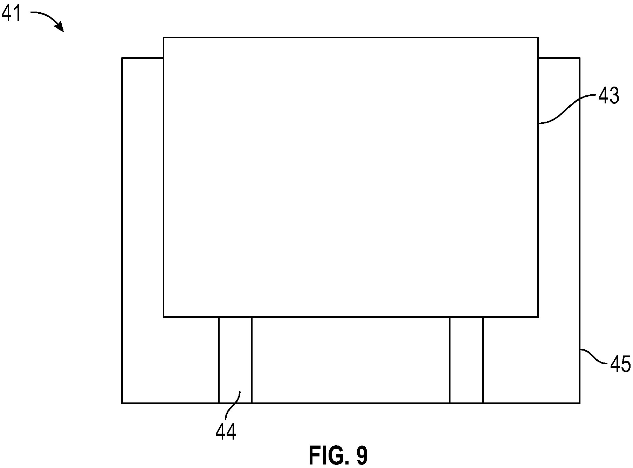

Referring to Figure 9, each pad 41 has a movable section 43 to hold the basket 42 that is connected to an electronic spring 44 below. The movable section 43 and the spring 44 are held together in a fixed section or housing 45 of the pad 41 which is made of polymer or any similar material. The electronic springs 44 of each pad 41 are connected to the control system 11

Whenever a change of weight is detected by the electronic springs 44 software in the control system 11 automatically calculate the difference between the latest recorded weight and the new recorded weight. The calculated difference is equal to the weight of food having been removed. To calculate the retail price, it multiplies the net weight removed from the shelf 3 by the price allocated to the location where the produce was removed. A printer may be connected to the control system 11 to immediately print the price of the produce removed.

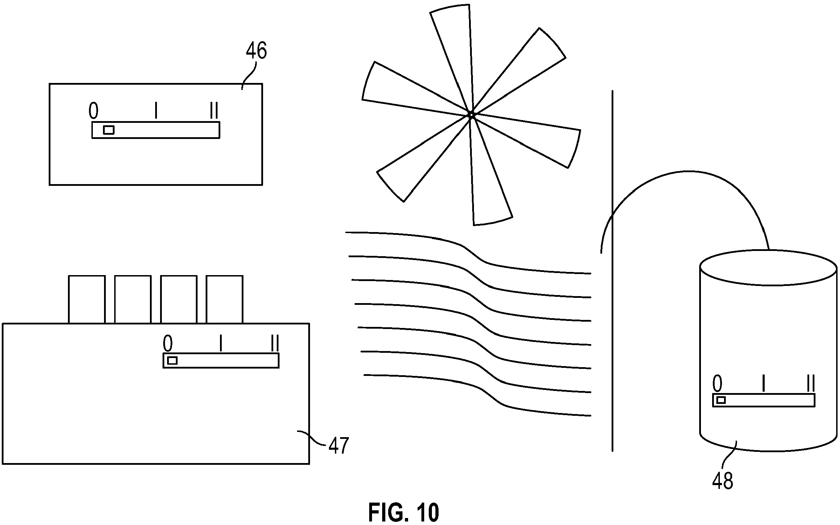

Figure 10 shows control units 46, 47, 48 for the ozone generator 10, humidifier 8 and cooling system 9. The control units 46, 47, 48 for the ozone generator 10, humidifier 8 and cooling system 9 are all designed on the same principles. Each control unit 46, 47, 48 can be set to at least two levels of ozone concentration, humidity concentration and temperature to be chosen based on the produce stored in the cabinet 1 to maximize shelf life of the stored produce. Each control unit 46, 47, 48 also has an "off" setting. The control units 46, 47, 48 form part of the control system 11.

The control system 11 constantly monitors the relative humidity, temperature and ozone level due to readings received from the sensors 36, 37, 38 and switches different parts of the apparatus 1 on and off when required. If the level of humidity, ozone or temperature in the cabinet 1 drops below, say, a pre-set level, then the relevant part would be automatically switched on to bring the level of ozone, humidity and/or temperature within their pre-set range. The ultrasonic humidifier 8 and ozone generator 10 may need to be run through much of the day as customers are likely to be opening the cabinet 1 frequently which would affect the environment inside the cabinet. Fresh air will be received into the cabinet 1 by a customer opening the cabinet 1. The control system 11 uses software that can identify the requirement of different types of produce and automatically sets the best environment in the cabinet 1 for maximising the shelf life of the produce being stored. The density of ozone and humidity can be changed to be at different levels at different periods (e.g. night and day). The control system 11 also monitors the level of water in a water tank supplying water to the ultrasonic humidifier 8 to see if it needs refilling. It can also send notifications regarding any issues or broken-down systems so that staff can be immediately notified and can quickly fix the issue or move the fresh produce to another cabinet to maintain a good conservation level.

The control system 11 is connected to the outside air inlet pump for pumping air into the cabinet 1 if the air is too rich in ozone or too cold and requires a rapid dilution.

The cabinet 1 has a power box 49 (see Figure 6) that is connected via a mains plug to the mains electricity to draw energy from the mains network. The power box 49 then distributes the power to the cooling system 9 which includes the condenser 22 and fan 20, the ultrasonic humidifier 8 and its internal fan, the ozone generator 10 and a fan associated with it, the humidity, ozone and temperature sensors or probes 36, 37, 38 and the display lights.

The cabinet 1 may have a back-up battery system to keep the cabinet 1 going for a limited amount of days in case of power outage.

The cabinet 1 may have a built-in timer to differentiate the night period from the day period. The timer may form part of the control system 11.

The cabinet 1 may include cameras in the display area which are used to assess the freshness and ripeness of produce. An infra-red camera may be used to assess precisely the level of ethylene being emitted by the produce. Based on these observations, the level of humidity, temperature and ozone can be adjusted to maximize the shelf life of produce in real time.

The cabinet 1 may be fitted with a screen that monitors when each variety of produce has been restocked. The screen may also be used for marketing purposes.

The cabinet 1 may have a card reader for a retailer personally to log in when each variety of food was restocked and how much weight of food was restocked. A card for the card reader may enable the retailer to draw all the information regarding the control environment data over a period of time (say the last month).

The cabinet 1 may be connected to a central data management system enabling a close monitoring of the amount of food on display. This permits a high degree of precision in maintaining stock levels and can be used to predict when to re-order produce based on instantaneous demand. Data transmission for this may be done remotely as the cabinet 1 may be connected to a WIFI network.

The cabinet 1 may have an audio sensor for reacting to consumers walking past the cabinet 1 to let them know about new arrivals, season produce or temporary sales.

The cabinet 1 may have a sensor for counting the number of people walking past and a sensor for noting the number of people that open the cabinet 1 to enable a ratio to be calculated.

The cabinet 1 may include a reverse osmosis system to purify the water being supplied to the ultrasonic humidifier 8 for health reasons and also for improving the operational life of the humidifier 8. The reverse osmosis system is connected to the water network by a hose through which it receives water from the water network. The reverse osmosis system is connected to the ultrasonic humidifier 8 by a hose to deliver the purified water to the humidifier 8.

The display area 2 of the cabinet 1 may include UV lighting which can be controlled by the control system 11. For example, the control system 11 may arrange for how long the UV lighting is to be on for and at what intensity it is to be on for.

The cabinet 1 is shown as a vertical, free standing cabinet but a cabinet could be a horizontal display cabinet. The vertical cabinet may have a set of six doors that can be opened separately or simultaneously. The doors are divided into three pairs of vertical doors that open away from the interior of the cabinet. The horizontal cabinet may be fitted with sets of doors that slide outwards or inwards to give access to the stored produce.

The cabinet 1 can be of different dimensions to suit the requirements of the customer.

The material used for the external shell of the cabinet 1 may be steel, stainless steel, aluminium or any other suitable metal and/or plastic, reinforced plastic or other any strong polymer. Materials for other parts of the cabinet 1 may be formed of stainless steel, aluminium, brass, food-safe silicone, rubber, polyurethane, polycarbonate, or any other suitable materials.

The method of manufacturing the cabinet 1 may make use of injection moulding, rotational moulding, compression moulding, die casting, sheet metal stamping/pressing, laser cutting, 3D printing, or any other suitable variations known in the art.

In a specific example of a preferred embodiment, the cooling system 9 is set to achieve one of two pre-set temperature ranges which are between 2-4°C and between 4-6°C. The cabinet 1 is arranged to provide a level of ozone in a range of 0.5 to 2 ppm for berries and grapes, 1.5 to 5 ppm for larger fruits, and 2 to 8 ppm for vegetables.

The cabinet 1 is arranged to provide a relative humidity level in a range of 90 to 100% for fresh vegetables, and 85 to 90% for fresh fruit.

At night (say 2 hours after the closing time of a retailer's shop) until morning (say 3 hours before opening time), the ozone generator 10 can be arranged to produce large amount of ozone to increase its concentration emitted from the shelves 3.

During the night or when the cabinet 1 is not opened by customers, the ozone generation system, the ultrasonic humidifier 8 and the cooling system 9 may automatically switch to a stand-by mode while settings are maintained within set limits.

The shelves 3 have an angle of tilt down towards the front of 2°.

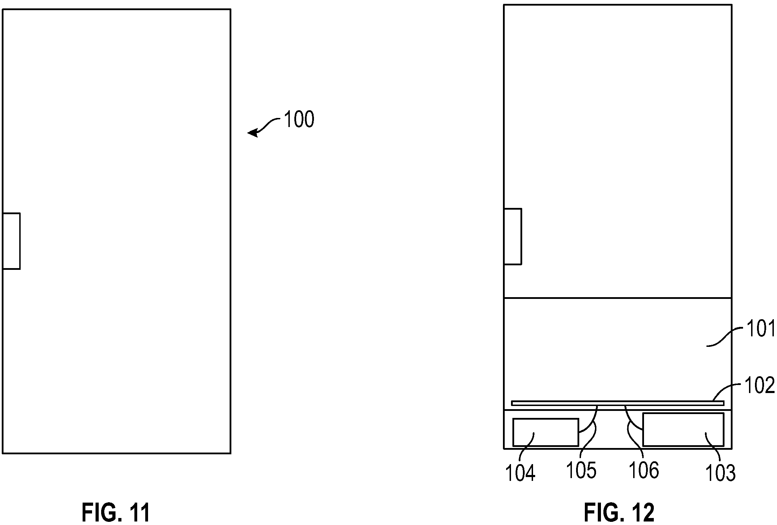

Referring to Figures 11 and 12, an apparatus 100 according to a second embodiment of the invention is shown forming part of a personal refrigerator. The personal refrigerator 100 is fitted with a lower section 101 forming a storage area where fresh produce can be stored. This lower section 101 can be fitted with shelving if required. The lower section 101 provides a controlled environment where the temperature, ozone and relative humidity is monitored and controlled. The bottom of this section 101 is fitted with a propagation piping network 102 composed of a cool air and ozone piping system and a separate humidity piping system similar to that previously described. Beneath the lower section 101 at the bottom of the refrigerator are located the humidifier 103 and the ozone generator 104. Pipes are made of polymers like PVC. The cool air and ozone piping system or network 105 distributes the ozone to the storage area while humidity piping system or network 106 distributes the humidity to the storage area.

Like the apparatus 1 for the first embodiment only electricity and water access are required for running this controlled environment section.

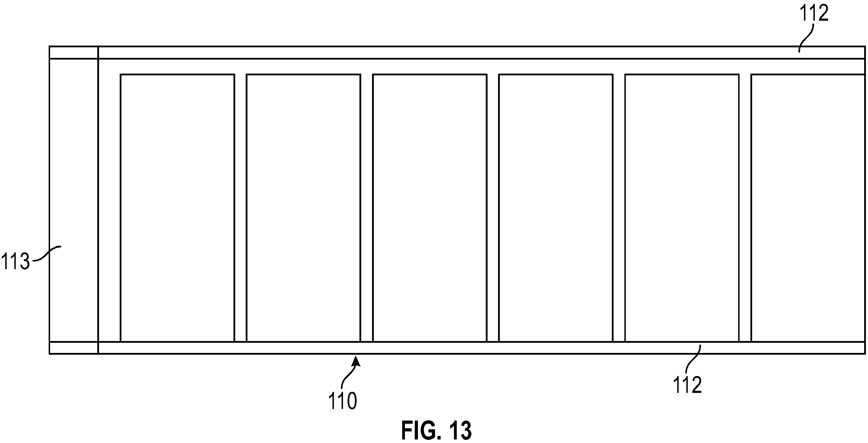

Referring to Figure 13, an apparatus 110 according to a third embodiment of the invention is shown forming part of a container for transporting or storing fresh produce. The produce is stored in boxes or pallets or any other holder that is partially open in the storage area 111 of the container 110. The container 110 has a piping network area 112 for a piping network used for the propagation of ozone and cooled air and a piping network used for the propagation of humidity on both sides of the storage area 111. The container 110 also has a support section 113 where the humidifier, ozone generator and cooling systems are located similar to that previously described.

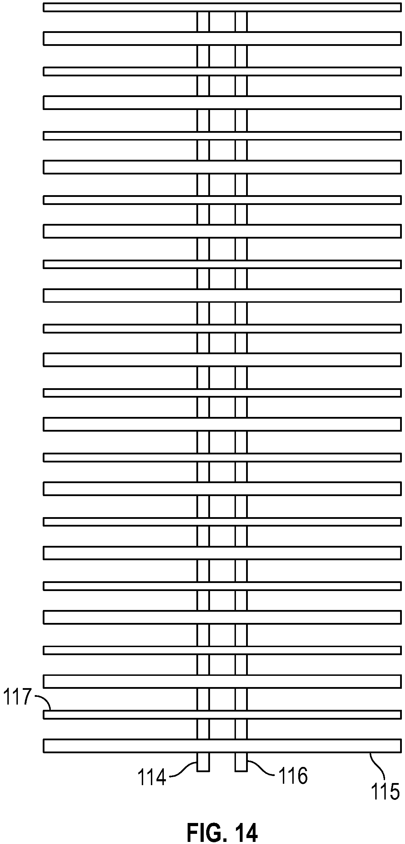

Referring to Figure 14, the cool air and ozone piping network comprises a main distribution pipe 114 connected to propagation pipes 115 (with holes) extending away from the main distribution pipe 114 to optimise propagation through the container 110. The humidity piping network comprises a main distribution pipe 116 connected to propagation pipes 117 (with holes) extending away from the main distribution pipe 116 to optimise propagation through the container 110.

Whilst particular embodiments have been described, it will be understood that various modifications may be made without departing from the scope of the claimed invention.

## Claims

1. An apparatus for preservation of fresh items or produce, said apparatus comprising a closed housing (1) to enable the environment within the housing to be controlled, one or more shelves (3) within the housing for supporting fresh items, an ozone generator (10), a humidifier (8) and a propagation or distribution system (23, 16) for transferring ozone generated by the ozone generator and water particles produced by the humidifier separately to the vicinity of the or each shelf before releasing both the ozone and water particles into the housing.

2. The apparatus as claimed in claim 1, wherein the housing (1) contains a plurality of shelves (3) for supporting fresh items, and the propagation or distribution system (23, 16) transfers ozone and water particles separately to each shelf for release in the vicinity of each shelf.

3. The apparatus as claimed in claim 1 or 2, wherein the propagation or distribution system (23, 16) comprises a plurality of pipes (24, 17) extending from the ozone generator (10) and the humidifier (8) to the or each shelf (3).

4. The apparatus as claimed in claim 3, wherein the plurality of pipes comprises first and second main pipes (24, 17) connected at one end thereof to the ozone generator (10) and humidifier (8) respectively.

5. The apparatus as claimed in claim 4, wherein each main pipe (24, 17) has a plurality of subsidiary pipes (32, 29) branching from its other end to distribute ozone and water particles in the vicinity of the shelf (3).

6. The apparatus as claimed in any preceding claim, including a closed loop air recirculation system (14) including air inlet conduits (13) connected to a cooling system (9) for cooling recirculated air.

7. The apparatus as claimed in claim 6 when dependent on claim 4, wherein the first main pipes (24) are connected to the ozone generator (10) and cooling system (9).

8. The apparatus as claimed in claim 6 or 7, including an automated control system (11) for controlling operation of the humidifier (8), the ozone generator (10) and the cooling system (9).

9. The apparatus as claimed in claim 8, wherein the or each shelf (3) has at least one of a humidity sensor (36), an ozone sensor (37) and a temperature sensor (38) connected to the control system (11), wherein the control system is arranged to adjust automatically the operation of the humidifier (8), the ozone generator (10), and the cooling system (9) in response to signals from said sensor or sensors.

10. The apparatus as claimed in any one of claims 6 to 9, wherein the closed housing (1) has a storage area (2) containing the one or more shelves (3) and a support area (6) separate from the storage area in which the humidifier (8), cooling system (9) and ozone generator (10) are located.

11. The apparatus as claimed in any preceding claim, including a weighing mechanism (41) on the or each shelf (3) to weigh the items stored on the shelf and to detect a change in weight when one or more items are removed from the shelf.

12. The apparatus as claimed in any preceding claim, including an infrared camera configured to assess the level of ethylene emitted from the items on the or each shelf (3).

13. The apparatus as claimed in any preceding claim, including a water tank connected to the humidifier (8) for containing water to be supplied to the humidifier, wherein the water tank is slidable from the closed housing (1) to outside of the closed housing to enable it to be refilled with water.

14. A method for preserving fresh items comprising the steps of:-
storing the fresh items on one or more shelves (3) in a closed housing (1);
feeding ozone and humidity separately from each other to the vicinity of the or each shelf (3); and
releasing the ozone and humidity into the closed housing (1) in said vicinity.

15. The method as claimed in claim 14, including one or more of the steps of:-
weighing items stored on the or each shelf (3) so as to detect a change in weight when one or more items are removed from the shelf;
circulating cooled air around the interior of the closed housing (1);
sensing at least one of the humidity, ozone and temperature levels at the or each shelf (3) and automatically adjusting the levels to provide a desired environment in the closed housing (1);
automatically changing the levels of humidity and ozone released in the vicinity of the or each shelf (3) in accordance with determined time periods and/or times of day and night;
illuminating the fresh items stored on the or each shelf (3) with UV lighting; and
using an infra-red camera to assess the level of ethylene being emitted by the items stored on the or each shelf (3) to adjust the required levels of humidity, ozone and/or temperature in the vicinity of the shelf.
